# EUROPÄISCHE PATENTSCHRIFT

(11) **EP 0 885 392 B1**
(45) Veröffentlichungstag und Bekanntmachung des Hinweises auf die Patenterteilung: **06.02.2002**
(21) Anmeldenummer: 97920514.3
(22) Anmeldetag: 03.03.1997
(51) Int. Cl.: G01N 33/569, A61K 38/19, A61K 39/395

(54) **DIFFERENTIALDIANOSTISCHES VERFAHREN ZUM NACHWEIS VERSCHIEDENER STADIEN DER ALZHEIMER-KRANKHEIT**
PROCESS FOR DIFFERENTIAL DIAGNOSIS OF VARIOUS STATES OF ALZHEIMER'S DISEASE
PROCEDE POUR LE DIAGNOSTIC DIFFERENTIEL DE DIFFERENTS STADES DE LA MALADIE D'ALZHEIMER

(30) Priorität: 06.03.1996 DE 19608519; 14.08.1996 DE 19632671; 20.09.1996 DE 19640348
(43) Veröffentlichungstag der Anmeldung: 23.12.1998
(73) Patentinhaber: Hartwig, Manfred, 13125 Berlin (DE); Schulz, Jörg, 13127 Berlin (DE)
(72) Erfinder: Hartwig, Manfred, 13125 Berlin (DE); Schulz, Jörg, 13127 Berlin (DE)
(74) Vertreter: Baumbach, Friedrich, Dr.
(86) Internationale Anmeldenummer: DE9700522
(87) Internationale Veröffentlichungsnummer: WO9733175

(56) Entgegenhaltungen:
- ALZHEIMER'S RESEARCH, Bd. 1, 1995, Seiten 137-140, XP002034793 M. HARTWIG ET AL.: "Accelerated apoptosis of cultured lymphocytes in Alzheimer's disease." in der Anmeldung erwähnt
- CLINICAL IMMUNOLOGY AND IMMUNOPATHOLOGY, Bd. 75, Nr. 3, 1995, Seiten 246-250, XP002034786 F. SHALIT ET AL.: "T lymphocyte subpopulations and activation markers correlate with severity of Alzheimer's disease." in der Anmeldung erwähnt
- MOLECULAR NEUROBIOLOGY, Bd. 9, 1994, Seiten 73-81, XP002034787 V. K. SINGH: "Studies of neuroimmune markers in Alzheimer's disease." in der Anmeldung erwähnt
- PROC. NATL. ACAD. SCI. USA, Bd. 93, 30.April 1996, Seiten 4147-4152, XP002034788 J. A. LONDON ET AL.: "Neurocytopathic effects of beta-amyloid-stimulated monocytes: A potential mechanism for central nervous system damage in Alzheimer disease."
- BRAIN, BEHAVIOR AND IMMUNITY, Bd. 10, Juni 1996, Seiten 115-125, XP002034789 E. W. P. NIJHUIS ET AL.: "Differences in dexamethasone-sensitivity between lymphocytes from patients with Alzheimer's disease and patients with multi-infarct dementia."
- AMERICAN JOURNAL OF PATHOLOGY, Bd. 150, Nr. 1, Januar 1997, Seiten 119-131, XP002034790 M. M. TOMPKINS ET AL.: "Apoptotic-like changes in Lewy-body-associated disorders and normal aging in substantia nigral neurons."
- ADVANCES IN NEUROIMMUNOLOGY, Bd. 6, Nr. 2, 1996, Seiten 191-222, XP002034791 J. ZIELASEK ET AL.: "Molecular mechanisms of microglial activation."

## Beschreibung

Die Erfindung betrifft ein differentialdiagnostisches Verfahren zum Nachweis verschiedener Stadien der Alzheimer-Krankheit sowie eine Verwendung von Makrophagen-Stimulationsfaktoren und eine Verwendung von Stoffen zur Herstellung eines Mittels zur Therapie. Anwendungsgebiete der Erfindung sind die Medizin und die pharmazeutische Industrie.

Die Alzheimer-Erkrankung ist die häufigste Form des senilen Schwachsinns. Sie führt über beginnende Verschlechterungen der mentalen Funktionen und eine pathologische Persönlichkeitsveränderung kontinuierlich zu einem Abbau der Gesamtpersönlichkeit mit erheblicher Einbuße der Lebensqualität, Pflege- und Betreuungsbedürftigkeit und schließlich nach 6-8 Jahren nach Erkennung der Erkrankung zum Tode.

Die Häufigkeit der Krankheit zeigt deutlich steigende Tendenz und läßt eine erhebliche Zunahme der Betreuungskosten erwarten. Statistische Erhebungen zeigen, daß 5% der über 70jährigen und 20% der über 80jährigen von dieser Krankheit befallen werden. Exakte, wissenschaftlich begründete bzw. beweisende Zahlen über Erkrankungshäufigkeit und Pflege- bzw. Betreuungsaufwendungen liegen nur unvollständig vor, so daß von einer noch höheren Krankheitsrate ausgegangen werden kann. In den USA gibt es derzeit ca. 4 Millionen erfaßte Alzheimer-Fälle, eine Verdreifachung dieser Anzahl bis zum Jahr 2050 wird vorhergesagt, da sich die Lebenserwartung weiter steigern wird. Die finanzielle Gesamtbelastung infolge notwendiger Betreuungs- und Pflegekosten sowie Bereitstellung von Hilfsmitteln wird in den USA im Jahre 1994 mit 80-100 Milliarden Dollar angegeben, so daß die durch die Alzheimer-Krankheit bedingten Ausgaben nach Herz-Kreislauferkrankungen und Krebs an dritter Stelle stehen.

Charakteristische neuropathologische Merkmale der Erkrankung sind extraneuronale, unlösliche Proteinablagerungen (senile Plaques), intraneuronale Knäuel verdrehter Proteinfäden (neurofibrilläre Bündel) und neuronale Degeneration. Trotz einer Reihe bekannter Risikofaktoren (wie fortgeschrittenes Alter, genetische Veranlagung oder ein Immundefekt) ist die auslösende Ursache der Krankheit bisher nicht bekannt.

Die definitive Diagnose der Erkrankung kann erst nach dem Tode anhand der histopathologischen Veränderungen im Gehirn gestellt werden. Die klinische Diagnose einer Alzheimer-Erkrankung wird gegenwärtig durch Anwendung und Beurteilung einer Vielzahl von anamnestischen, klinischen, laborchemischen, psychometrischen und aufwendigen bildgebenden Untersuchungsverfahren gestellt. Dabei ist jedoch stets nur eine wahrscheinliche, aber nicht beweisende Zuordnung der Untersuchungsergebnisse möglich. Bei Anwendung aller dieser Methoden unter der Voraussetzung fachkompetenter und geriatrisch-neurologisch erfahrener Beurteilung ist eine 85-90%ige Diagnosesicherheit im Vergleich zum post mortem erhobenen histopathologischen Befund möglich.

Neue diagnostische Möglichkeiten werden im Zusammenhang mit dem vermehrten Vorkommen des sog. E4-Allels des Apolipoprotein E-Gens bei Alzheimer-Kranken (Rebeck et al., Neuron 11 (1993) 575) gesehen. Auch veränderte Immunparameter wurden auf ihr diagnostisches Potential hin angesprochen (Singh, Molec. Neurobiol. 9 (1994) 73; Shalit et al., Clin. Immunol. Immunopathol. 75 (1995) 246), aber nicht systematisch untersucht. Veränderte zelluläre Kaliumkanäle bei der Alzheimer-Demenz (Fortschr. Med. 112 (1994) 6, 14) blieben bisher den Beweis ihrer diagnostischen Tauglichkeit unter klinischen Bedingungen und im Frühstadium der Erkrankung schuldig.

Einfache und bessere Diagnoseverfahren mit hoher Treffsicherheit können dazu beitragen, daß bei dem Verdacht auf das Vorliegen einer Alzheimer-Erkrankung routinemäßig eine deutlich geringere Anzahl von Diagnosekriterien zur Anwendung kommen kann, eine breite Anwendung insbesondere auch im ambulanten Gesundheitswesen ermöglicht, eine frühzeitige Zuweisung in spezialisierte Einrichtungen realisierbar und die therapeutische Sicherheit hinsichtlich Anwendung kurativer Verfahren bei unterschiedlichen Formen der Demenz deutlich erhöht wird. Daraus dürften infolge einer möglichen frühzeitigeren Intervention erhebliche Vorteile für den Betroffenen als auch für die pflegenden Angehörigen sowie für die Gesamtgesellschaft resultieren.

Die Entwicklung von Therapeutika wird insbesondere durch das Fehlen adäquater Tiermodelle behindert. Zur Zeit stehen lediglich Medikamente zur Verfügung, die mit der Krankheit verbundene Defizite zu lindern versuchen, eine entscheidende Verbesserung oder gar Heilung jedoch nicht bewirken können. Eine Vielzahl von Pharmaka wird heute angeboten, die von unterschiedlichen pathophysiologischen Prämissen ausgehen. So werden rheologisch wirksame Produkte (z. B. Gingko-biloba-Präparate), Nootropika, Kalziumantagonisten und weitere Substanzen verordnet, ohne daß kausale Wirkungsmechanismen nachweisbar sind. Alle bisherigen therapeutischen Bemühungen hatten einen symptomatischen Hintergrund.

In den letzten Jahren wurden Cholinesterasehemmer intensiv untersucht. Eines dieser Medikamente ist Tacrin, das jedoch Nebenwirkungen aufweist (u. a. Leberschädigungen) und auch nur vorübergehend wirkt. Über 200 Verbindungen sind weltweit bereits getestet worden, 14 Verbindungen befinden sich in den USA in klinischen Tests. Man schätzt, daß Medikamente zur Erleichterung der Erkrankung in den nächsten 5-10 Jahren zur Verfügung stehen werden, daß es Medikamente zur Heilung jedoch frühestens in 15 Jahren geben wird (Altmann, Molecular medicine today 1 (1995) 9, 404).

Das Ziel der Erfindung ist es, ein neues und empfindliches diagnostisches Verfahren zu finden, das eine einfache und periphere Diagnose verschiedener Stadien der Krankheit ermöglicht, ohne dabei den Patienten wesentlich zu belasten sowie eine weitere Verwendung von an sich bekannten Stoffen und Faktoren zur Herstellung von Arzneimitteln zu finden, deren Wirkungen gegen die auslösende Ursache der Alzheimer-Krankheit gerichtet sind und die damit für eine Prophylaxe und eine Therapie dieser Erkrankung oder von humanen Erkrankungen analoger Ursache geeignet sind.

Ausgangspunkt der Erfindung ist der überraschende Befund, daß sich der pathologische Alzheimer-Prozeß nicht nur im Gehirn, sondern auch spezifisch im peripheren Blut manifestiert. Daraus abgeleitet war die Aufgabe der Erfindung, ein differentialdiagnostisches Verfahren zu finden, das wesentliche Seiten des Alzheimer-Prozesses im Gehirn durch den Nachweis der analogen Reaktionen im peripheren Blut charakterisiert.

Ein wesentliches und charakteristisches Merkmal der Alzheimer-Krankheit ist die chronische Aktivierung und dramatische Vermehrung der gehirnresidenten Mikrogliazellen zu reaktiven Gehirnmakrophagen in Reaktion auf ein bisher unbekanntes auslösendes Agens. Der äquivalente Prozeß im peripheren Blut ist eine chronische Aktivierung und Vermehrung von Monozyten bzw. der aus ihnen differenzierenden Makrophagen, die durch gesteigerte Phagozytose (Aufnahme und Abbau) apoptotischer Zellen deren pathologisch erhöhter Produktion entgegenwirkt. Im letzten, schweren Stadium III der Erkrankung bricht der Prozeß der Phagozytose apoptotischer Zellen zunehmend zusammen.

Die Erfindung wird durch ein differentialdiagnostisches Verfahren gemäß den Ansprüchen 1 und durch Verwendung von natürlichen, rekombinanten oder synthetischen Makrophagen-Stimulationsfaktoren oder ihren biologisch aktiven Untereinheiten bzw. modifizierten Formen davon zur Herstellung eines Arzneimittels zur Therapie der Alzheimer-Krankheit gemäß den Ansprüchen 2 bis 5 realisiert und durch Verwendung eines Stoffes, der selektiv an Aktivierungsmarker oder Adhäsionsrezeptoren auf aktivierten T-Lymphozyten oder an Adhäsionsrezeptoren auf Endothelzellen bindet, zur Herstellung eines Mittels zur Therapie der Alzheimer-Krankheit gemäß den Ansprüchen 6 bis 8.

Mit dem Verfahren gemäß Anspruch 1 wird im Probanden-blut die Zahl der apoptotischen Zellen im Vergleich zu den entsprechenden Normalwerten bei gesunden, etwa gleichaltrigen Personen bestimmt. Zur Realisierung des Verfahrens sind sämtliche herkömmlichen Methoden geeignet, die dem quantitativen Nachweis apoptotischer Zellen dienen, wie z. B. der immunhistochemische Nachweis von Apoptose-Markern oder die Durchfluß-Zytometrie.

Für die zusätzliche Bestimmung der Aktivierung von Monozyten/Makrophagen bei Probanden im Vergleich zu den entsprechenden Normalwerten bei gesunden, etwa gleichaltrigen Personen. Zur Realisierung dieser Variante sind sämtliche herkömmlichen Methoden geeignet, die die Aktivierung von Monozyten/Makrophagen messen, wie z. B. ihre Vermehrung, ihre Adhäsion an Plasteoberflächen, ihre Aufnahmekapazität für Testpartikel, die Expression von Aktivierungs- und Proliferationsmarkern oder die Sekretion von Aktivierungs- und Proliferationsfaktoren.

Wenn sich die gemessenen Parameter im Verlauf der Erkrankung nicht kontinuierlich ändern, so steigt z. B. der Anteil der adhärenten Monozyten/Makrophagen im I. (leichten) und II. (mittelgradigen) Stadium der Erkrankung an und fällt dann zum III. (schweren) Stadium wieder ab. Die Unterscheidung zwischen Stadium I und III wird dann erfindungsgemäß mittels der parallelen Bestimmung des Anteils apoptotischer Zellen möglich.

Mit der Anwendung des Verfahrens steht eine neue und relativ einfache differentialdiagnostische Methode für den Nachweis verschiedener Stadien der Alzheimer-Krankheit zur Verfügung, die bekannte Methoden im Hinblick auf spezifische Aspekte der Erkrankung ergänzt und eine frühzeitige Erkennung und Einteilung in verschiedene Stadien zuläßt. Es ist ein wesentlicher Vorteil dieses Verfahrens, daß es nicht nur eine qualitative Diagnostik, sondern durch die Abhängkeit der gemessenen Blutparameter vom Schweregrad der Alzheimer-Demenz auch eine quantitative Diagnostik sowie Verlaufskontrollen ermöglicht. In Fällen einer reinen vaskulären Demenz sprechen bekannte Diagnose-Verfahren erwartungsgemäß nicht an. Allerdings weisen klinisch diagnostizierte vaskuläre Demenzen post mortem im histopathologischen Befund häufig zusätzlich Alzheimer-Merkmale auf (Jellinger, Acta Neuropathol. 91 (1996) 219).
Erfindungsgemäß ist auf einfache Weise eine Differentialdiagnostik gegen die reine vaskuläre Demenz möglich.

Ausgehend von dem überraschenden Befund, daß ein auslösendes Prinzip der Erkrankung die pathologische Ansammlung absterbender (apoptotischer) Lymphozyten im peripheren Blut und im Gehirn ist, war eine weitere Aufgabe der Erfindung, eine Verwendung von Stoffen zur Herstellung eines Arzneimittels zu finden, das einer pathologischen Ansammlung von apoptotischen Lymphozyten im Körper, insbesondere im Gehirn, verhindert und damit der Auslösung der Alzheimer-Krankheit entgegenwirkt.

Diese Aufgabe wird erfindungsgemäß durch eine Verwendung von Makrophagen-Stimulationsfaktoren zur Herstellung eines Arzneimittels gemäß den Ansprüchen 2 bis 5 gelöst. Dafür wurden Faktoren verwendet, die die Phagozytose-Aktivität im Körper, insbesondere im Gehirn, steigern. Diese Steigerung wird entweder
- durch verstärkte Bildung von Makrophagen aus Vorläuferzellen, wie Monozyten oder Mikroglia-Zellen,
- durch Vermehrung von Makrophagen oder deren Vorläuferzellen,
- durch eine Steigerung der Phagozytose-Aktivität von Makrophagen oder
- durch ihr gesteigertes Überleben bewirkt.

Demzufolge sind zur Realisierung der Erfindung sämtliche Makrophagen-Stimulationsfaktoren geeignet, d. h. alle natürlichen, rekombinanten oder synthetischen Faktoren oder ihre biologisch aktiven Untereinheiten bzw. modifizierte Formen davon. Spezielle Ausführungsformen der Erfindung sind Verwendungen von Makrophagen-Stimulationsfaktoren zur Herstellung eines Arzneimittels, die den humanen oder humanen rekombinanten Makrophagen-Monozyten chemotaktischen und aktivierenden Faktor (M-CAF), den humanen oder humanen rekombinanten Makrophagen-Kolonie-stimulierenden Faktor (M-CSF) oder den humanen oder humanen rekombinanten Granulozyten-Makrophagen-Koloniestimulierenden Faktor (GM-CSF) enthalten.

Die Verwendung der Mittel erfolgt gegebenenfalls mit an sich üblichen pharmazeutschen Hilfs-, Träger- und Zusatzstoffen, bevorzugt in isotonischer wäßriger Lösung. Die Applikation erfolgt in geeigneten Formen, wie beispielsweise oral, intravenös, in Form implantierbarer Minipumpen oder analoger Depotformen. Ein relativ niedriges Molekulargewicht des verwendeten Wirkstoffs erlaubt das Passieren der Blut-Hirn-Schranke, insbesondere wenn diese im Alter und bei der Alzheimer-Krankheit eine erhöhte Durchlässigkeit besitzt. Bekanntermaßen können auch Schleppersubstanzen, wie z.B. Nanopartikel, angekoppelt werden, die eine optimale Passage durch die Blut-Hirn-Schranke ermöglichen. Eine zusätzliche Wirkung der eingesetzten Faktoren besteht darin, im Hirn von Alzheimer-Kranken den Abbau seniler Plaques durch Gehirnmakrophagen zu fördern. Die Verträglichkeit der erfindungsgemäß eingesetzten Faktoren ist gut, was am Beispiel der Anwendung von GM-CSF bei Knochenmark-Transplantationen gezeigt werden kann.

Eine wesentliche an sich bekannte Anwendungsform von pharmazeutischen Mitteln besteht darin, den als Wirkstoff dienenden Faktor erst im Körper entstehen zu lassen. Bevorzugt ist dabei die Implantation von gentechnisch modifizierten Zellen, die diesen Faktor produzieren. In Betracht kommt auch die Implantation von Reservoirs, die den Faktor kontinuierlich abgeben.

Ausgehend von dem überraschenden Befund, daß ein auslösendes Prinzip der Alzheimer-Erkrankung die pathologische Produktion aktivierter, autospezifischer T-Lymphozyten im peripheren Blut ist, die nach Passage der Blut-Hirn-Schranke bei Wechselwirkung mit gehirnresidenten Mikroglia-Zellen der Apoptose unterliegen und so die für die Krankheit spezifische Aktivierung der Mikroglia induzieren, verbunden mit einem chronischen neurotoxischen Entzündungsprozeß, war eine weitere Aufgabe der Erfindung, eine Verwendung von Stoffen zur Herstellung eines Mittels zur Therapie der Alzheimer-Krankheit zu finden, die eine Wechselwirkung aktivierter T-Lymphozyten mit den Mikroglia-Zellen im Gehirn verhindert. Diese Aufgabe wird erfindungsgemäß dürch eine Verwendung eines Stoffes, der selektiv an Aktivierungsmarker oder Adhäsionsrezeptoren auf aktivierten T-Lymphozyten oder an Adhäsionsrezeptoren auf Endothelzellen bindet, zur Herstellung eines Mittels zur Therapie der Alzheimer-Krankheit gemäß den Ansprüchen 6 bis 8 gelöst.

Der erfindungsgemäß verwendete Stoff bindet selektiv an aktivierte T-Lymphozyten oder ihre endothelialen Adhäsionspartner. Die aktivierten T-Lymphozyten können auf diese Weise in ihrer Adhäsion zum Endothel blockiert (Variante 1), durch Bindung zu diesem Stoff entfernt (Variante 2) oder mittels des gebundenen Stoffes abgetötet (Variante 3) werden.

Die Variante 1 besteht darin, Antikörper oder lösliche Formen von Gegenrezeptoren einzusetzen, die gegen Adhäsionsrezeptoren auf aktivierten T-Lymphozyten oder gegen Adhäsionsrezeptoren auf Endothelzellen gerichtet sind, diese Strukturen blockieren und damit die Adhäsion der aktivierten T-Lymphozyten am Endothel als notwendigen Schritt für die Passage der Blut-Hirn-Schranke verhindern. Spezielle Ausführungsformen dieser Variante sind monoklonale Antikörper gegen die Adhäsionsrezeptoren LFA-1 oder VLA-4 sowie gegen ihre Gegenrezeptoren ICAM-1, ICAM-2 und VCAM-1 oder lösliche Formen dieser Rezeptoren oder ihrer Gegenrezeptoren. Die bevorzugte Anwendung dieser Wirkstoffe erfolgt intravenös in isotoner wässriger Lösung. Für die Antikörper werden humane oder humanisierte Antikörper bevorzugt.

Die Variante 2 besteht darin, aktivierte T-Lymphozyten durch Bindung zu entfernen. Dazu sind Antikörper sowie Gegenrezeptoren für Aktivierungsmarker oder Adhäsionsrezeptoren auf aktivierten T-Lymphozyten geeignet. Spezielle Ausführungsformen sind monoklonale Antikörper gegen den IL-2-Rezeptor (IL-2R), gegen den invarianten Teil des Histokompatibilitätsmarkers HLA-DR oder gegen die Adhäsionsrezeptoren LFA-1 oder VLA-4. Geeignet sind auch das IL-2-Molekül als Substrat des IL-2-Rezeptors sowie die Gegenrezeptoren ICAM-1, ICAM-2 und VCAM-1. Spezielle Anwendungsformen bestehen darin, die Antikörper oder Gegenrezeptoren auf festen Strukturen zu immobilisieren oder sie als Vermittler für die Bindung zu festen Strukturen (s. Anwendungsbeispiel) zu benutzen und die gebundenen aktivierten T-Lymphozyten dann nach dem Prinzip einer In-Vitro-Blutwäsche zu entfernen.

Zur Realisierung der Variante 3 sind Antikörper geeignet, die gegen Aktivierungsmarker oder Adhäsionsrezeptoren auf aktivierten T-Lymphozyten gerichtet sind und mittels Komplementaktivierung die aktivierten T-Lymphozyten abtöten. Spezielle Ausführungsformen sind monoklonale Antikörper, die gegen den invarianten Teil des Aktivierungsmarkers HLA-DR, gegen den IL-2-Rezeptor oder gegen die Adhäsionsrezeptoren LFA-1 oder VLA-4 auf aktivierten T-Lymphozyten gerichtet sind. Die bevorzugte Anwendung dieser Antikörper erfolgt in isotoner wässriger Lösung als intravenöse Gabe. Bevorzugt wird auch die Anwendung humaner oder humanisierter monoklonaler Antikörper.

Die Erfindung läßt sich natürlich auch mit bindenden Untereinheiten der eingesetzten Stoffe realisieren.

Mit der Anwendung der erfindungsgemäßen Verwendung von Faktoren und Stoffen zur Herstellung von Arzneimitteln steht eine neue Methode zur Behandlung der Alzheimer-Krankheit zur Verfügung. Die erfindungsgemäße Verwendung hat den Vorteil, daß sie gegen die Ursache der Krankheit wirkt und damit eine Progression der Erkrankung verhindert.

Die Erfindung wird nachfolgend an Ausführungsbeispielen näher erläutert.

### Beispiel 1

### Allgemeine Methodik

Dem Probanden wird eine Probe venösen Bluts entnommen, um damit Parameter gemäß Anspruch 1 zu bestimmen.

### Spezielle Ausführung

10 ml venöses Blut in Citratpuffer als Antikoagulant werden in bekannter Weise dazu benutzt, um die mononuklearen Zellen zu isolieren. Im unmittelbaren Anschluß daran wird unter diesen einerseits der prozentuale Anteil apoptotischer Zellen mittels Durchfluß-Zytometrie und andererseits der prozentuale Anteil von Zellen mittels Zellzählung bestimmt, der nach einstündiger Inkubation der Zellsuspension bei 37 °C in Plaste-Kulturplatten durch Adhärenz zurückbleibt.

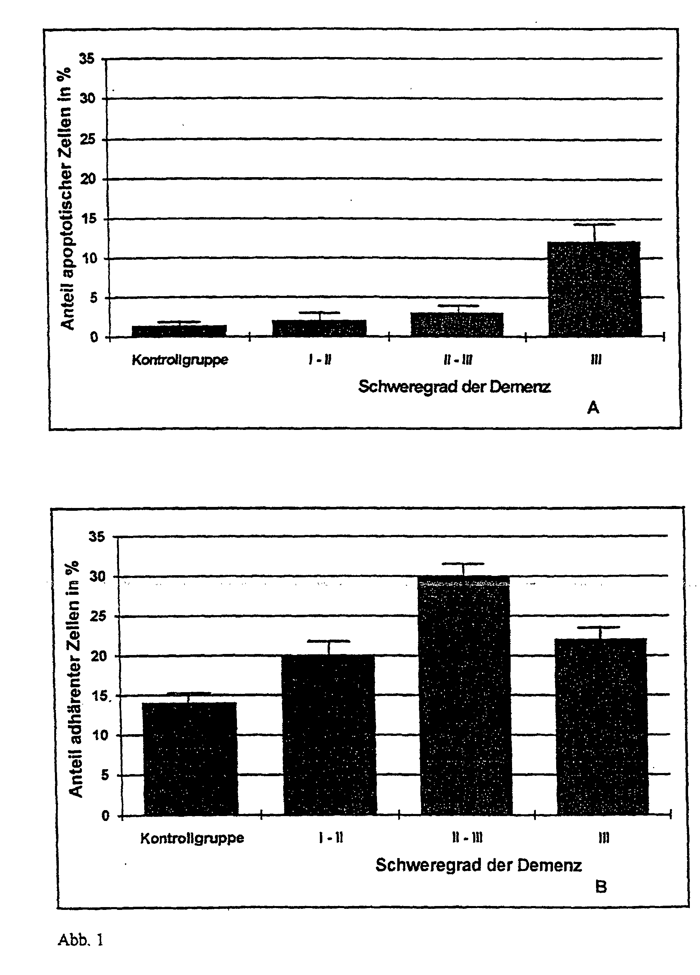

Ein Vergleich der Ergebnisse für verschiedene, nach Alters- und Geschlechtszusammensetzung vergleichbare Personengruppen:
1. Kontrolle (Mittleres Alter: 74 Jahre; n = 15)
2. Alzheimer-Demenz (Mittleres Alter: 76 Jahre)
   Stadium I - II, n = 10; Stadium II - III, n = 10; Stadium III, n = 10
3. Vaskuläre Demenz (Mittleres Alter: 75 Jahre; n = 15, alle Stadien gemischt)
zeigt für die Alzheimer-Kranken im Vergleich zur Kontrollgruppe eine mit dem Schweregrad der Demenz zunehmende Akkumulation apoptotischer Zellen, die im Stadium III beträchtlich ist (Abb. 1A). Als Reaktion darauf wächst der Anteil adhärenter Zellen im Stadium I-II und II-III signifikant an, um im Stadium III wieder abzunehmen (Abb. 1B).

In der Gruppe der vaskulären Demenzen ist zwar die Zahl der adhärenten Zellen mit 18% gegenüber der Kontrollgruppe mit 14% erhöht, jedoch nicht die Zahl der apoptotischen Zellen.

### Beispiel 2

### Allgemeine Methodik

Die Wirkung der Faktoren wird in einer Kultur von mononuklearen Zellen des peripheren Bluts geprüft, die im Falle von Alzheimer-Patienten mit einer anormalen Ansammlung apoptotischer Lymphozyten den äquivalenten Prozeß im Gehirn und damit eine wesentliche auslösende Ursache der Alzheimer-Krankheit widerspiegelt.

### Spezielle Ausführung

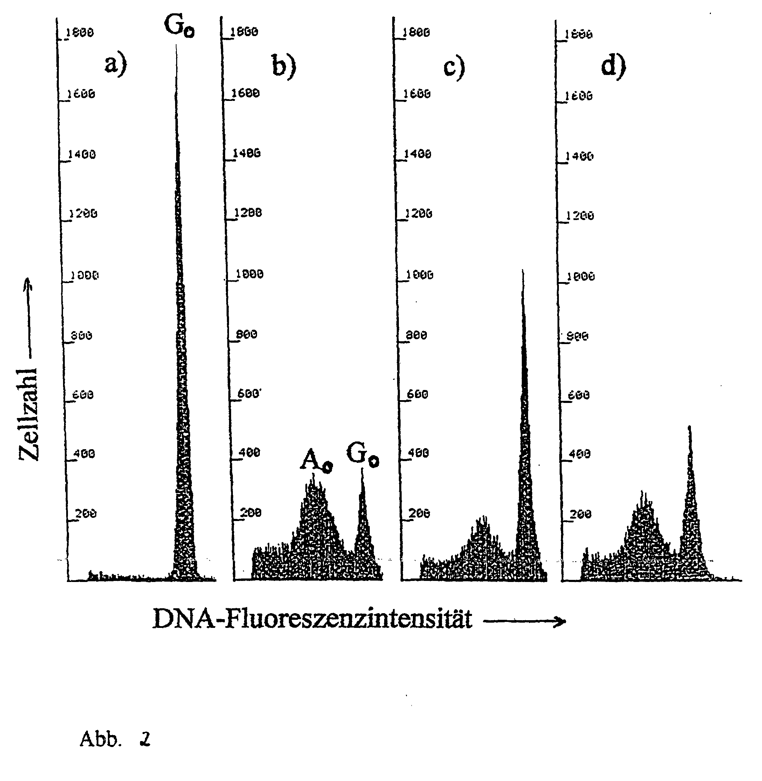

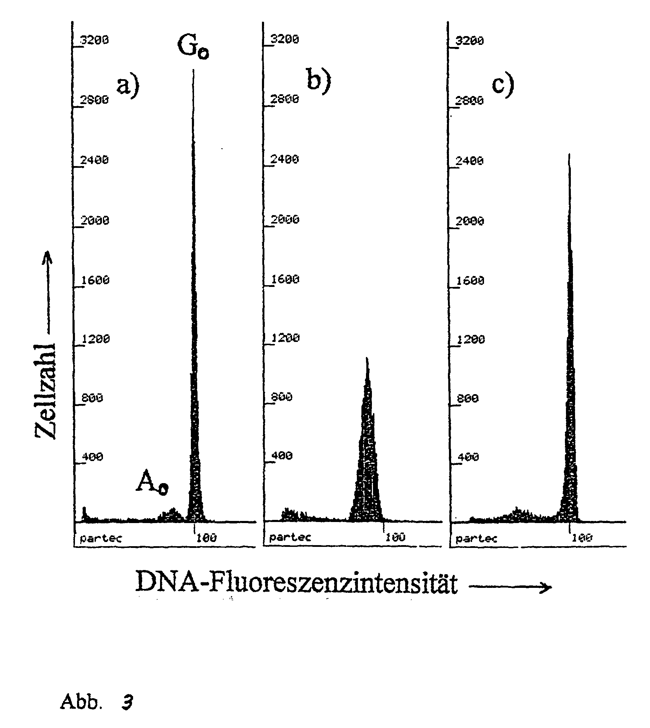

Venöses Blut von Alzheimer-Patienten wird in bekannter Weise dazu benutzt, um eine Kultur mononuklearer Zellen zu etablieren und deren Gehalt an apoptotischen Lymphozyten mit Hilfe der Durchflußzytometrie zu bestimmen (Literatur: Hartwig et al, Alzheimer's Res. 1 (1995) 137). In Gegenwart des humanen Granulocyten-Makrophagen-Kolonie-stimulierenden Faktors (hGM-CSF; rekombinantes Produkt aus E. coli; Boehringer Mannheim, Deutschland) ist der Gehalt an apoptotischen Lymphozyten in der Zellkultur verringert, wie an dem relativen Anteil der sog. Aₒ-Fraktion abgelesen werden kann, die die apoptotische Zellpopulation repräsentiert (Abb. 2). Die Konzentrationsabhängigkeit der Wirkung zeigt, daß die Phagozytose-Aktivität von Monozyten/Makrophagen gegenüber apoptotischen Lymphozyten optimal bei niedrigen Konzentrationen des Faktors, d. h. im ng/ml-Bereich, stimuliert wird. Zu einem ähnlichen Ergebnis kommt der Phagozytose-Test.

### Beispiel 3

### Allgemeine Methodik

Die Wirkung des erfindungsgemäßen Mittels wird in einer Kultur von mononuklearen Zellen des peripheren Bluts geprüft, die im Falle von Alzheimer-Patienten mit einer anormalen Produktion apoptotischer Zellen eine wesentliche auslösende Ursache der Alzheimer-Krankheit widerspiegelt.

### Spezielle Ausführung

Venöses Blut von Alzheimer-Patienten wird in bekannter Weise dazu benutzt, um eine Kultur mononuklearer Zellen zu etablieren und deren Gehalt an apoptotischen Zellen mit Hilfe der Durchflußzytometrie zu bestimmen (Literatur: Hartwig et al, vgl. Beispiel 2). Aktivierte T-Lymphozyten werden vor der Kultivierung abgetrennt, indem zunächst der von ihnen exprimierte IL-2-Rezeptor (IL-2R) mit monoklonalen IL-2R-Antikörpern (Maus IgG1; Boehringer Mannheim) besetzt wird und anschlieBend die so markierten Zellen mittels Dynabeads (M-450 Ratte anti-Maus IgGl; Dynal, Oslo) biomagnetisch separiert werden.

Die nach 72h Kultur erhaltenen DNA-Histogramme (Abb. 3) für die mononuklearen Zellen zeigen für die gesunde Kontrolle (a) den typischen Gₒ-Peak für ruhende Zellen (bei der willkürlich gewählten MaBzahl 100) und links davon die sogenannte Aₒ-Region, die den Anteil apoptotischer Zellen anhand ihres partiellen DNA-Verlustes charakterisiert. Während für die Kontrolle der Anteil apoptotischer Zellen gering ist, sind im Falle des Alzheimer-Patienten (b) fast alle Zellen nach 72h Kultur apoptotisch. Die vorherige biomagnetische Separation aktivierter T-Lymphozyten hemmt dagegen den apoptotischen Prozeß (c) und damit eine wesentliche auslösende Ursache der Alzheimer-Krankheit.

### Legende zu den Abbildungen

### Abb. 1

Prozentualer Anteil apoptotischer (A) und adhärenter mononuklearer Zellen (B) im peripheren Blut gesunder Kontrollpersonen und von Alzheimer-Patienten in Abhängigkeit vom Schweregrad der Demenz. Fehlerbalken zeigen die Standardabweichung des Mittelwertes.

### Abb. 2:

DNA-Histogramme für 72h kultivierte mononukleare Zellen des peripheren Bluts von (a) einer gesunden Kontrollperson und (b-d) von einem Alzheimer-Patienten.
a) Kontrolle, erwartungsgemäB fast alle Zellen in Gₒ des Zellzyklus
b) Patient, 0 ng/ml hGM-CSF, hoher Anteil apoptotischer Zellen in Ao
c) Patient, 1 ng/ml hGM-CSF, Anteil apoptotischer Zellen in Aₒ deutlich verringert
d) Patient, 5 ng/ml hGM-CSF, Anteil apoptotischer Zellen in Aₒ verringert, aber weniger deutliche Wirkung als bei 1 ng/ml.

### Abb. 3

DNA-Histogramme für mononukleare Zellen des peripheren Bluts nach 72h Kultur
a) Gesunde Kontrollperson
b) Alzheimer-Patient, Stadium III
c) Patient wie unter b), jedoch nach biomagnetischer Separation aktivierter T-Lymphozyten

## Patentansprüche

1. Differentialdiagnostisches Verfahren zum Nachweis verschiedener Stadien der Alzheimer-Krankheit,
**dadurch gekennzeichnet, daß**
der Anteil apoptotischer Zellen und der Anteil adhärenter Monozyten und Makrophagen in Proben peripheren Bluts parallel bestimmt und mit den Normalwerten verglichen wird, wobei bei Vorliegen des Stadiums I und II bei zunehmender Akkumulation apoptotischer Zellen der Anteil der adhärenten Monozyten/Makrophagen ansteigt und bei Vorliegen des Stadiums III der Anteil der adhärenten Monozyten/Makrophagen fällt.

2. Verwendung von natürlichen, rekombinanten oder synthetischen Makrophagen-Stimulationsfaktoren oder ihren biologisch aktiven Untereinheiten bzw. modifizierten Formen davon zur Herstellung eines Arzmeimittels zur Therapie der Alzheimer-Krankheit.

3. Verwendung nach Anspruch 2, **dadurch gekennzeichnet, daß** der humane oder humane rekombinante Makrophagen-Monozyten chemotaktische und aktivierende Faktor (M-CAF) eingesetzt wird.

4. Verwendung nach Anspruch 2, **dadurch gekennzeichnet, daß** der humane oder humane rekombinante Makrophagen-Koloniestimulierende Faktor (M-CSF) eingesetzt wird.

5. Verwendung nach Anspruch 2, **dadurch gekennzeichnet, daß** der humane oder humane rekombinante Granulozyten-Makrophagen-Kolonie-stimulierende Faktor (GM-CSF) eingesetzt wird.

6. Verwendung eines Stoffes, der selektiv an Aktivierungsmarker oder Adhäsionsrezeptoren auf aktivierten T-Lymphozyten oder an Adhäsionsrezeptoren auf Endothelzellen bindet, zur Herstellung eines Mittels zur Therapie der Alzheimer-Krankheit.

7. Verwendung nach Anspruch 6, **dadurch gekennzeichnet, daß** Antikörper, immobilisierte Antikörper, eine Kombination von Antikörpern oder Gegenrezeptoren eingesetzt werden.

8. Verwendung nach Anspruch 6, **dadurch gekennzeichnet, daß** Antikörper gegen Aktivierungsmarker oder Adhäsionsrezeptoren auf aktivierten T-Lymphozyten oder gegen Adhäsionsrezeptoren auf Endothelzellen eingesetzt werden.

## Claims

1. Differential diagnostic method for the detection of various stages of the Alzheimer disease
wherein
the share of apoptotic cells and the share of adherent monocytes and macrophages in samples of peripheral blood is determined in parallel and compared with the standard values, with the share of adherent monocytes/macrophages increasing in the existence of stage I and II with an increasing accumulation of apoptotic cells and the share of adherent monocytes/macrophages dropping in the existence of stage III.

2. Use of natural, recombinant or synthetic macrophage stimulating factors or their biologically active sub-units or modified forms thereof for the production of a medication for the therapy of the Alzheimer disease.

3. Use according to Claim 2, wherein chemotactical human or human recombinant macrophage colony activating factor (M-CAF) is used.

4. Use according to Claim 2, wherein human or human recombinant macrophage colony stimulating factor (M-CSF) is used.

5. Use according to Claim 2, wherein human or human recombinant granulocyte macrophage colony stimulating factor (GM-CSF) is used.

6. Use of a material which binds selectively to activation markers or adhesion receptors on activated T-lymphocytes or to adhesion receptors on endothelium cells for the production of an agent for the therapy of the Alzheimer disease.

7. Use according to Claim 6, wherein antibodies, immobilised antibodies, a combination of antibodies or anti-receptors are used.

8. Use according to Claim 6, wherein antibodies against activation markers or adhesion receptors on activated T-lymphocytes or against adhesion receptors on endothelium cells are used.

## Revendications

1. Procédé de diagnostic différentiel permettant d'identifier différents stades de la maladie d'Alzheimer, se caractérisant par le fait que la proportion en cellules apoptotiques et la proportion en monocytes et de macrophages adhérents dans des échantillons de sang périphérique soient déterminées parallèlement et comparées avec les valeurs normales, en considérant qu'en présence des stades I et II en présence d'une accumulation croissante de cellules apoptotiques la proportion en monocytes/macrophages adhérents augmente et qu'en présence du stade III, la proportion des monocytes/macrophages adhérents se réduit

2. Emploi de facteurs de stimulation des macrophages qui soient naturels, de recombinaison ou synthétiques ou de leurs sous-unités bio-actives resp. leurs formes modifiées pour la fabrication d'un médicament dans l'objectif de traiter la maladie d'Alzheimer.

3. Emploi selon la revendication 2, se caractérisant par le fait que le facteur chimiotactique et activateur de monocytes-macrophages humains ou de recombinaison humaine (M-CAF) soit utilisé.

4. Emploi selon la revendication 2, se caractérisant par le fait que le facteur stimulateur de colonies de macrophages humains ou de recombinaison humaine (M-CSF) soit utilisé.

5. Emploi selon la revendication 2, se caractérisant par le fait que le facteur stimulateur de colonies de macrophages granulocytes humains ou de recombinaison humaine (GM-CSF) soit utilisé.

6. Emploi d'une substance qui lie par sélection des marqueurs d'activation ou des récepteurs d'adhésion à des lymphocytes T activés ou des récepteurs d'adhésion à des cellules endothéliales, pour la fabrication d'un médicament dans l'objectif de traiter la maladie d'Alzheimer.

7. Emploi selon la revendication 6, se caractérisant par le fait que des anticorps, des anticorps immobilisés, une combinaison d'anticorps ou d'anti-récepteurs soient utilisés.

8. Emploi selon la revendication 6, se caractérisant par le fait que des anticorps soient utilisés contre des marqueurs d'activation ou des récepteurs d'adhésion sur des lymphocytes T activés ou contre des récepteurs d'adhésion sur des cellules endothéliales.
